# EUROPÄISCHE PATENTSCHRIFT

(11) **EP 0 613 660 B1**
(45) Veröffentlichungstag und Bekanntmachung des Hinweises auf die Patenterteilung: **15.01.1997**
(21) Anmeldenummer: 94101344.3
(22) Anmeldetag: 29.01.1994
(51) Int. Cl.: A61B 17/28

(54) **Medizinische Zange**
Surgical forceps
Pince chirurgicale

(30) Priorität: 24.02.1993 DE 9302650 U
(43) Veröffentlichungstag der Anmeldung: 07.09.1994
(73) Patentinhaber: Karl Storz GmbH & Co., D-78532 Tuttlingen (DE)
(72) Erfinder: Lang, Dieter, D-96342 Stockheim (DE)
(74) Vertreter: Weller, Wolfgang, Dr.rer.nat.

(56) Entgegenhaltungen:
- WO-A-89/11827
- DE-U- 9 115 760

## Beschreibung

Die Erfindung betrifft eine medizinische Zange, mit zwei relativ zueinander bewegbaren Griffelementen am patientenfernen Ende, durch die zwei bewegbar am patientennahen Ende angeordnete Maulteile über ein Betätigungselement relativ zueinander bewegbar sind, und bei der beide Maulteile gemeinsam, ohne Relativbewegung zueinander, verschwenkbar sind.

Eine derartige medizinische Zange gemäß dem Oberbegriff des Anspruchs 1 ist aus der WO-A-89 11 827 bekannt. Die bekannte medizinische Zange, die in Verbindung mit einem Bronchoskop zur Entnahme von Gewebe aus der Luftröhre oder den Bronchien dient, weist am patientennahen Ende zwei Maulteile auf, die zum Abtrennen des Gewebes relativ zueinander bewegbar sind, d.h. geöffnet und geschlossen werden können. Zwischen einem Zangenschaft und den Maulteilen ist ein verschwenkbares Element angeordnet, dessen patientenfernes Ende über ein Scharniergelenk mit dem Zangenschaft verbunden ist, und dessen patientennahes Ende die beiden gelenkigen Maulteile trägt, so daß die beiden Maulteile sowohl relativ zueinander beweglich als auch gemeinsam aus der Längsachse des Zangenschaftes heraus verschwenkbar sind.

Ein spritzenartiges Griffelement dient zur Betätigung der Öffnungs- und Schließbewegung der beiden Maulteile, während mit einem weiteren Betätigungselement das verschwenkbare Element betätigt wird. Das Öffnen und Schließen der beiden Maulteile einerseits und das gemeinsame Verschwenken der beiden Maulteile andererseits sind bei der bekannten Zange voneinander unabhängige Vorgänge und erfordern jeweils eine eigene getrennte Bedienung durch den Benutzer.

Eine weitere medizinische Zange ist aus dem deutschen Gebrauchsmuster G 91 15 760.9 bekannt. Eines der beiden Maulteile ist als starres Maulteil ausgebildet, dessen Ausrichtung derart ist, daß es mit einer Röhre, die ein Griffelement am patientenfernen Ende mit dem starren Maulteil am patientennahen Ende verbindet, einen spitzen Winkel einschließt, der zum patientenfernen Ende öffnet.

Das zweite Maulteil, das relativ zum ersten bewegbar ist, bewegt sich bei der Öffnungsbewegung der Maulteile in Richtung der Röhre.

Nachteilig an der zuvor erwähnten medizinischen Zange ist, daß sie, trotz möglichst geringer Dimensionierung der Bauteile am patientennahen Ende durch die hakenförmige Ausbildung des starren Maulteils recht sperrig baut.

Ein möglicher Einsatzzweck einer derartigen medizinischen Zange besteht darin, in eine menschliche Kieferhöhle eingeführt zu werden, um darin ein Stück Gewebe, beispielsweise eine Zyste, zu entfernen. Das Einführen des patientennahen Endes der medizinischen Zange in die Kieferhöhle kann durch die Nasenöffnung und eine in der Nasenwand vorgesehene Öffnung, ein sogenanntes Fenster, erfolgen, wozu bei einem solchen Eingriff in aller Regel eine örtliche Betäubung ausreichend ist, und somit keine aufwendige Operation mit Vollnarkose unter Öffnung der Kieferhöhle von der Außenseite her notwendig ist.

Durch die abgewinkelte Anordnung der Maulteile ist es zwar möglich, die Maulteile an die zu entfernende Zyste anzulegen und diese durch die geöffneten Maulteile zu fassen, der Abtrennvorgang selbst ist jedoch relativ schwierig durchzuführen. Nach dem Fassen der Zyste durch die Maulteile wird diese durch eine Bewegung der gesamten Zange mehr oder weniger abgerissen. Darüber hinaus ist es äußerst schwierig, aufgrund der hakenartigen Ausbildung der Zange am patientennahen Ende, diese wieder aus der Kieferhöhle abzuziehen, da dabei die Gefahr besteht, daß sie sich mit anderen Gewebeteilen in unerwünschter Weise verhakt.

Aufgabe der vorliegenden Erfindung ist daher, eine medizinische Zange der eingangs genannten Art derart zu verbessern, daß das an Ort und Stelle bringen der Zange sowie der Abtrennvorgang des Gewebes verbessert und erleichtert wird.

Erfindungsgemäß wird die Aufgabe dadurch gelöst, daß über das Betätigungselement in einem ersten Bewegungsbereich der Griffelemente beide Maulteile gemeinsam verschwenkbar sind, und daß in einem zweiten Bewegungsbereich der Griffelemente ein Maulteil relativ zum anderen Maulteil bewegbar ist.

Diese Maßnahmen haben den erheblichen Vorteil, daß in dem ersten Bewegungsbereich der Griffelemente die beiden Maulteile zusammen, d.h. beispielsweise geschlossen aneinanderliegend, bewegt werden können. Diese Bewegbarkeit der geschlossenen Maulteile ermöglicht es nun, diese am patientennahen Ende der Zange in eine solche Stellung zu bewegen, die für das an Ort und Stelle bringen des patientennahen Endes am günstigsten ist. Nimmt man beispielsweise den eingangs erwähnten Einführvorgang in die Kieferhöhle heran, so können die Maulteile geschlossen aneinanderliegend in eine Stellung gebracht werden, in der diese am günstigsten und beim Patienten am wenigsten Beeinträchtigungen erzeugend, also am patientennahen Ende schlank bauend, in die Nase eingeschoben werden können. Ist eine Öffnung, ein sogenanntes Fenster, in der Nasenwand vorgesehen, durch das die Maulteile in die Kieferhöhle eingeführt werden sollen, können die geschlossenen Maulteile entweder in der Stellung, wie sie in die Nase eingeführt worden sind, oder durch ein entsprechendes Verschwenken in eine solch günstige Position gebracht werden, die ein Einführen in die Kieferhöhle erleichtern. Ist die Zange mit ihren Maulteilen in der Kieferhöhle an Ort und Stelle gebracht, beispielsweise an eine Stelle, an der eine Zyste abgetrennt werden muß, können die weiterhin geschlossenen Maulteile anschließend durch Drehen der gesamten Zange in eine Stellung gebracht werden, in der diese, beispielsweise wie bei dem eingangs erwähnten Gebrauchsmuster, in einer etwa hakenförmigen Abwinklung mit Öffnung zum patientenfernen Ende zu liegen kommen. Dadurch, daß in einem zweiten Bewegungsbereich der Griffelemente nun ein Maulteil relativ zum anderen Maulteil bewegbar ist, können diese Maulteile zum Öffnen und Fassen des abzutrennenden Gewebeteils, beispielsweise der Zyste, geöffnet und an diese herangelegt werden. Nach dem Ergreifen des Gewebeteils durch die Maulteile können diese durch entsprechend entgegengesetzt gerichtete Bewegung der Griffelemente wieder geschlossen werden und dabei das Gewebestück fest halten. Bei einer weiteren Bewegung der Griffelemente wirkt sich nun ein weiterer erheblicher Vorteil der Erfindung aus, nämlich, daß die Maulteile, die das Gewebestück ergriffen haben, im ersten Bewegungsbereich der Griffelemente beide zusammen bewegt bzw. verschwenkt werden können, so daß, ohne daß die gesamte medizinische Zange bewegt werden muß, lediglich die Griffelemente bewegt werden, beispielsweise geschlossen werden. Dabei bewegen sich die Maulteile gemeinsam von der Entnahmestelle weg, wodurch der Abtrennvorgang des Gewebes in vorteilhafter Weise unterstützt wird. Die Kombination der konstruktiven Maßnahme, nämlich Bewegbarkeit der Maulteile relativ zueinander und zugleich Verschwenkbarkeit der beiden Maulteile miteinander mit dem dazwischen ergriffenen Gewebeteil, erleichtern erheblich den Ergreif- und Abtrennvorgang des Gewebeteiles. Beim nachfolgenden Ausführen der medizinischen Zange aus dem Körper kommt der erhebliche Vorteil der Verschwenkbarkeit der beiden Maulteile zusammen dahingehend zum Ausdruck, daß diese Maulteile mit dem zwischen diesen erfaßten und abgetrennten Gewebeteil in eine solche Position bewegt bzw. verschwenkt werden können, die ein Abziehen der medizinischen Zange vom Körper erleichtert.

Somit wird die Aufgabe vollkommen gelöst.

In einer weiteren Ausgestaltung der Erfindung sind beide Maulteile um eine gemeinsame Achse verschwenkbar am patientennahen Ende angeordnet.

Diese Maßnahme hat nun den Vorteil, daß über eine geringe Anzahl an Bauteilen, nämlich die gemeinsame Achse, die beiden Maulteile verschwenkbar angeordnet sind, was zu einer raumsparenden und schlanken Bauweise am patientennahen Ende führt.

In einer weiteren Ausgestaltung der Erfindung ist im zweiten Bewegungsbereich der Griffelemente ein Maulteil blockiert, und das andere Maulteil ist relativ zum blockierten Maulteil bewegbar.

Diese Maßnahme hat nun ebenfalls den erheblichen Vorteil, daß durch einfache, wenig sperrende bauliche Maßnahmen der Übergang von der gemeinsamen Bewegung der verschwenkbaren Maulteile zu der Relativbewegung der Maulteile untereinander ermöglichst wird. Es wird lediglich ein Maulteil blockiert, beispielsweise durch einen Anschlag, so daß das andere Maulteil mittels desselben Betätigungsmechanismus wie bei der vorangegangenen Bewegung weiter bewegt werden kann.

In einer weiteren Ausgestaltung der Erfindung ist das Betätigungselement einerseits mit einem Griffelement verbunden und ist andererseits mit dem Maulteil gelenkig verbunden, das auch im zweiten Bewegungsbereich bewegbar ist.

Diese konstruktive Maßnahme hat nun den erheblichen Vorteil, daß durch lediglich ein einziges Betätigungselement dasjenige Maulteil bewegt wird, das sich gegenüber dem blockierten Maulteil weiter bewegt, dies aber auch zugleich bei der geimeinsamen Bewegung der Maulteile das andere Maulteil mitbewegen kann.

In einer weiteren Ausgestaltung der Erfindung ist das Maulteil, das im zweiten Bewegungsbereich ortsfest blockiert ist, im ersten Bewegungsbereich gegen die Kraft einer Feder von der Blockierstellung weg in Richtung gemeinsamer Verschwenkung der Maulteile bewegbar, und diese Federkraft unterstützt bei der entgegengesetzten Richtung die gemeinsame Bewegung beider Maulteile zusammen.

Diese Maßnahme hat nun den erheblichen Vorteil, daß, von der Blockierstellung in Richtung gemeinsamer Verschwenkung gerichtet, diese Bewegung des Maulteils gegen Federkraft durchgeführt werden muß, d.h. es ist ein ausreichender Anpreßdruck der beiden Maulteile während der gemeinsamen Verschwenkung vorhanden. Ein durch die Maulteile gefaßtes und abgetrenntes Gewebestück ist während dieser Bewegung andauernd fest und somit unverlierbar gehalten. In entgegengesetzter Richtung, d.h. bei der Verschwenkung der aneinanderliegenden Maulteile in Richtung der Stellung, in der eines der Maulteile blockiert wird, unterstützt die Federkraft positiv.

In einer weiteren Ausgestaltung der Erfindung ist das Maulteil, das ortsfest blockierbar ist, gelenkig mit einem mit einer Feder beaufschlagten Steuerelement verbunden, das an dem Griffelement beweglich gelagert ist, das nicht mit dem Betätigungselement verbunden ist.

Diese Maßnahme hat nun den erheblichen Vorteil, daß die die Federkraft erzeugenden Bauelemente abseits des patientennahen Endes im Bereich des patientenfernen Endes angeordnet werden können, wo wesentlich mehr Bauraum zur Verfügung steht, und lediglich über das Steuerelement die Federkraft auf das Maulteil am patiennahen Ende übertragen wird. Die Anordnung dieser Bauelemente an dem Griffelement, an dem nicht das Betätigungselement angeordnet ist, ermöglicht nunmehr eine vom Betätigungselement völlig unabhängige Einstellung der Federkraft an diesem anderen Griffelement, so daß eine für den jeweiligen Benutzer der medizinischen Zange optimale bzw. für diesen angenehme Federkraft einstellbar ist.

In einer weiteren Ausgestaltung der Erfindung ist das Steuerelement am patientenfernen Ende mit einem Endstück versehen, auf das die Feder einwirkt.

Diese Maßnahme hat nun den Vorteil, daß durch konstruktiv einfache und robuste Maßnahmen die zuvor erwähnte konstruktive Ausgestaltung der Federkraftbeaufschlagung bewerkstelligt wird, wodurch nicht nur die Handhabbarkeit, sondern auch die Betriebssicherheit der Zange erhöht wird.

In einer weiteren Ausgestaltung der Erfindung verläuft die Zange am patientennahen Ende gekrümmt.

Diese Maßnahme hat den Vorteil, daß durch die vorgesehene Krümmung für bestimmte Einsatzzwecke bereits eine ausgängliche Kontur am patientennahen Ende geschaffen wird, die für das Einführen in Körperöffnungen, beispielsweise in die Nasenhöhle, geeignet ist. Durch die zusätzliche Verschwenkbarkeit der Maulteile ist dann eine jeweils optimale Lageorientierung bzw. Krümmung und Abwinklung der Bauteile am patientennahen Ende individuell zu bewerkstelligen, so daß nicht nur dem Einsatzzweck, sondern auch den jeweiligen anatomischen Gegebenheiten des Patienten optimal angepaßt werden kann, somit das Ein- und Ausführen der Zange unter Anpassung an dessen Anatomie mit möglichst wenig Beeinträchtigungen für den Patienten durchführbar ist.

In einer weiteren Ausgestaltung der Erfindung ist die Zange integraler Bestandteil eines Endoskops.

Diese Maßnahme hat nun den Vorteil, daß in Zusammenwirkung mit dem Endoskop die jeweiligen örtlichen Gegebenheiten beobachtet und dadurch die optimale Lageorientierung der Maulteile bewerkstelligt werden kann.

Es versteht sich, daß die vorstehend genannten und die nachstehend noch zu erläuternden Merkmale nicht nur in der angegebenen Kombination, sondern auch in anderen Kombinationen sowie in Alleinstellung einsetzbar sind, ohne den Rahmen der vorliegenden Erfindung zu verlassen. Die Erfindung wird nachfolgend an Hand eines ausgeführten Ausführungsbeispieles näher beschrieben und erläutert. Es zeigen:
- Fig. 1: eine Seitenansicht einer erfindungsgemäßen medizinischen Zange in einer ersten Betriebsstellung mit geschlossenen Maulteilen,
- Fig. 2: eine detaillierte, ausschnittsweise, teilweise geschnittene Ansicht der medizinischen Zange von Fig. 1 im Bereich der Griffelemente,
- Fig. 3: eine noch weiter vergrößerte, ausschnittsweise, teilweise geschnittene Darstellung des patientennahen Endbereiches der medizinischen Zange von Fig. 1,
- Fig. 4: eine der Fig. 1 vergleichbare Darstellung der erfindungsgemäßen medizinischen Zange in einer zweiten Betriebsstellung mit verschwenkten, jedoch noch geschlossenen Maulteilen,
- Fig. 5: eine der Darstellung von Fig. 2 vergleichbare Darstellung in der zweiten Betriebsstellung,
- Fig. 6: eine der Fig. 3 vergleichbare Darstellung in der zweiten Betriebsstellung,
- Fig. 7: eine der Fig. 1 vergleichbare Darstellung der erfindungsgemäßen medizinischen Zange in einer dritten Betriebsstellung mit geöffneten Maulteilen,
- Fig. 8: eine den Fig. 2 und 5 vergleichbare Darstellung in der dritten Betriebsstellung, und
- Fig. 9: eine den Fig. 3 und 6 vergleichbare Darstellung in der dritten Betriebsstellung.

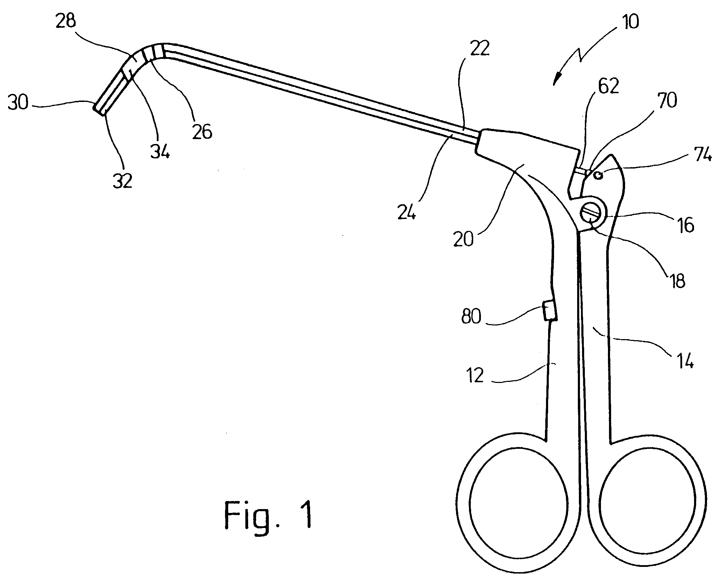

Eine in Fig. 1 dargestellte erfindungsgemäße medizinische Zange ist in ihrer Gesamtheit mit der Bezugsziffer 10 versehen.

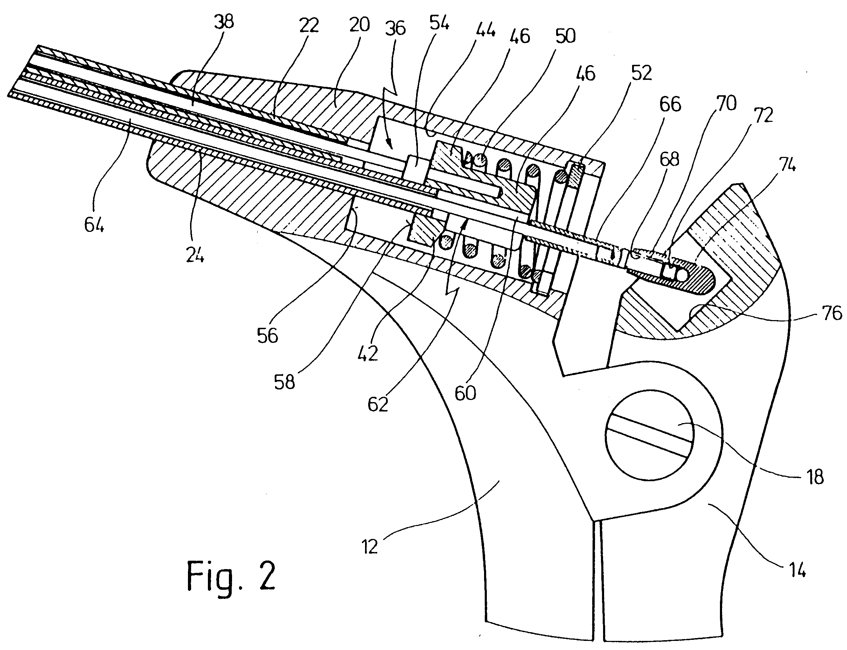

Die Zange 10 weist zwei Griffelemente 12 und 14 auf, die über ein Scharnier 16 miteinander verbunden sind. Die Verbindung erfolgt dabei mittels einer Schraube 18, wodurch die beiden Griffelemente 12 und 14 um die hier nicht näher dargestellte Längsmittelachse der Schraube 18 relativ zueinander verschwenkbar sind. Von einem oberen Endbereich 20 des Griffelementes 12 (siehe auch insbesondere Fig. 2) führen zwei eng aneinanderliegende Rohre 22 und 24 ab, von denen jedes etwa einen Außendurchmesser von 1,5 mm aufweist. Die Rohre können aus Stahl oder einem Kunststoffmaterial mit vergleichbaren Eigenschaften bestehen.

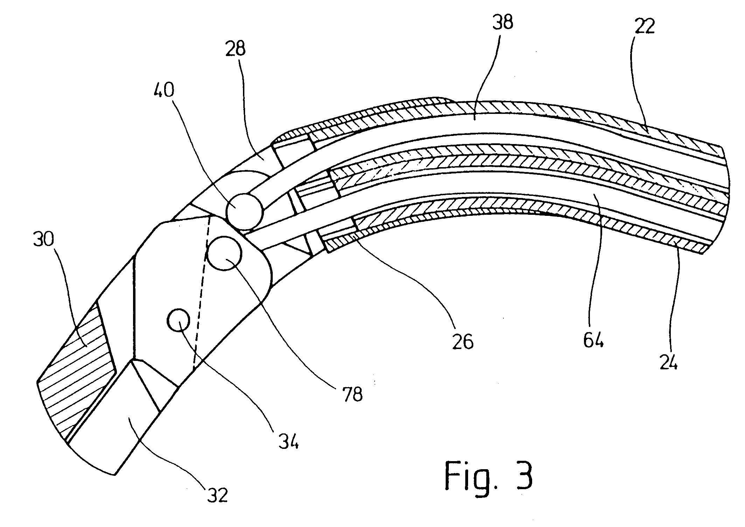

Am patientennahen Ende enden die beiden Rohre 22, 24 etwa auf gleicher Höhe (siehe insbesondere Fig. 3) und sind mit einem Endstück 26 verbunden, dessen äußeres Ende als Gabel 28 ausgebildet ist. Zwischen den beiden äußeren Zinken der Gabel 28 sind zwei Maulteile 30, 32 angeordnet, die über einen gemeinsamen Achszapfen 34 schwenkbar zwischen der Gabel 28 aufgenommen sind. Die Mittellängsachse des Achszapfens 34 stellt somit eine Schwenkachse der beiden Maulteile 30, 32 dar.

Das Maulteil 30 ist mit einem Steuerelement 36 in Form eines Drahtes 38 verbunden, und zwar mittels eines am Ende des Drahtes 38, quer zu dessen Längsrichtung verlaufenden Zapfens 40. Somit ist der Draht 38 etwa wie das Ende eines Bowdenzuges ausgebildet. Über den Zapfen 40 ist eine gelenkige Verbindung zwischen dem Ende des Drahtes 38 und dem Maulteil 30 geschaffen. Wie insbesondere aus Fig. 3 zu entnehmen, kommt der Zapfen 40 im Abstand zum Achszapfen 34 zum Liegen.

Der Draht 38 ist im Rohr 22 aufgenommen und ist an seinem dem Zapfen 40 gegenüberliegenden Ende mit einem Endstück 42 (siehe insbesondere Fig. 2) fest verbunden. Das Endstück 42 ist in einer Aussparung 44 im Endbereich 20 des Griffelementes 12 aufgenommen. Die Aussparung 44 ist dabei als Sacklochöffnung ausgebildet, in die das Endstück 42 einführbar ist.

Von einer Bodenfläche 56 der Aussparung 44 führt in Richtung patientennahes Ende ein hier nicht näher bezeichneter Durchtrittskanal, in dem die Rohre 22 und 24 angeordnet sind.

Das Endstück 42 weist eine hier nicht näher bezeichnete Sacklochbohrung auf, in der ein Endstück des Drahtes 38 aufgenommen ist, wobei der Draht 38 über eine Klemme 54 unverlierbar mit dem Endstück 42 verbunden ist.

Das Endstück 42 weist einen der Bodenfläche 56 der Aussparung 44 zugewandten Ringflanschabschnitt 46 auf, der in einen durchmessergeringeren zylindrischen Abschnitt 48 übergeht. Um den zylindrischen Abschnitt 48 ist eine Schraubenfeder 50 angeordnet, die sich auf der oberen Ringfläche des Ringflanschabschnittes 46 abstützt. Auf der gegenüberliegenden Seite stützt sich die Schraubenfeder 50 auf einem Sprengring 52 ab, der in einer hier nicht näher bezeichneten Ringnut im öffnenden Endbereich der Aussparung 44 eingesetzt ist.

Die Schraubenfeder 50 ist dabei derart vorgespannt, daß sie die Tendenz hat, das Endstück 42, in der in Fig. 2 dargestellten Position, in Richtung Bodenfläche 56 der Aussparung 44 zu drücken. Im Endstück 42 ist ferner eine durchgehende Bohrung 60 vorgesehen, durch die ein Betätigungselement 62 beweglich durchreicht.

Das Betätigungselement 62 weist einen Draht 64 auf, dessen patientenferner Endbereich mit einer Endhülse 66 fest verbunden ist, die über das, in der Darstellung von Fig. 2 rechte Ende des Griffelementes 12 vorsteht. Die Endhülse 66 ist mit einem Außengewinde 68 versehen und ist in eine mit einem Innengewinde 72 versehenen Aufnahmehülse 70 eingedreht. Die Aufnahmehüle 70 ist in einer Aussparung 76 am oberen Ende des Griffelementes 14 über eine Schraube 74 schwenkbar angebracht.

Der Draht 64 reicht von der Endhülse 66 durch die Bohrung 60 im Endstück 42 hindurch und ist durch das Rohr 24 bis zum Maulteil 32 geführt. (Siehe Fig. 3). An diesem patientennahen Ende ist der Draht 64 über einen Zapfen 78, der quer zu dessen Längsachse verläuft, gelenkig mit dem Maulteil 32 verbunden. Der Endbereich des Drahtes 64 ist dabei gleich wie der Endbereich des Drahtes 38, wie zuvor beschrieben, ausgebildet, der Zapfen 78 kommt dabei ebenfalls im Abstand zum Achszapfen 34, um den beide Maulteile 30, 32 verschwenkbar sind, zum Liegen.

Aus Fig. 1 ist zu entnehmen, daß am Griffelement 12 eine Stellschraube 80 vorgesehen ist, die eine Justierung der in Fig. 1 dargestellten ersten Betriebsstellung der medizinischen Zange 10 ermöglicht.

Aus Fig. 1 ist ferner zu entnehmen, daß die Zange 10 am patientennahen Ende, also im Bereich des Endstückes 26, gekrümmt verläuft und anschließend in die in dem dargestellten Ausführungsbeispiel geradlinig verlaufenden Maulteile 30, 32 übergeht.

Die Maulteile 30, 32 sind als geradlinig verlaufende etwa balkenförmige Teile mit nicht näher dargestellten aneinanderliegenden Schneiden ausgebildet. Wie nachfolgend noch erläutert, dienen die Maulteile dazu, ein Gewebeteil zu fassen, abzutrennen und das abgetrennte Gewebeteil zwischen den Maulteilen zu halten.

Zu diesem Zweck sind nun zahlreiche Ausgestaltungen der Maulteile 30, 32 möglich. Sie können als löffelartig übereinandergreifende Teile ausgebildet sein, sie können als messerartige Schneiden mit Überwurfteilen ausgebildet sein, es muß lediglich die Doppelfunktion, nämlich Abtrennen und Halten des Gewebeteils, möglich sein. Es ist ferner auch möglich, die Maulteile 30, 32 entsprechend der in Fig. 1 dargestellten Krümmung weiter gekrümmt fortzuführen um, je nach dem Einsatzzweck, ein möglichst einfaches Einführen des patientennahen Endbereiches durch eine Körperöffnung, beispielsweise durch eine Nasenöffnung eines Menschen, zu gewährleisten.

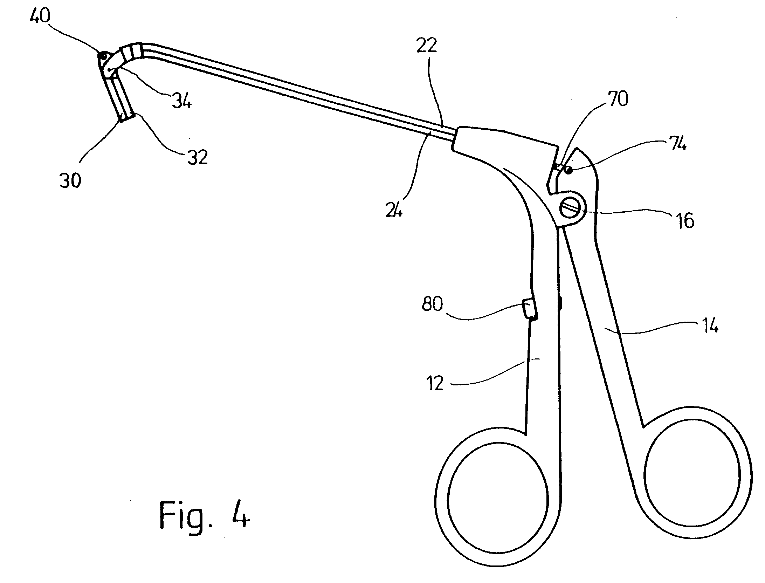

Aus der in den Fig. 1 bis 3 dargestellten ersten Betriebsstellung kann die Zange 10 durch Öffnen der Griffelemente 12 und 14 in die in Fig. 4 bis 6 dargestellte zweite Betriebsstellung gebracht werden.

Bei dieser Bewegung wird das Griffelement 14, in der Darstellung von Fig. 1 und 4, entgegen dem Uhrzeigersinn um das Scharnier 16 verschwenkt. Dabei wird der Draht 64 durch das Rohr 24 in Richtung patientennahes Ende verschoben und verschwenkt dabei das Maulteil 32 über die gelenkige Verbindung mittels des Zapfens 78 um den Achszapfen 34. Der Draht 38 folgt der Bewegung des Drahtes 64, wobei dies durch die Kraft der Schraubenfeder 50 unterstützt wird, d.h. bei dieser Bewegung wird über die Schraubenfeder 50 das Endstück 42 und somit auch der mit diesem fest verbundene Draht 38 ebenfalls synchron in Richtung patientennahes Ende verschoben.

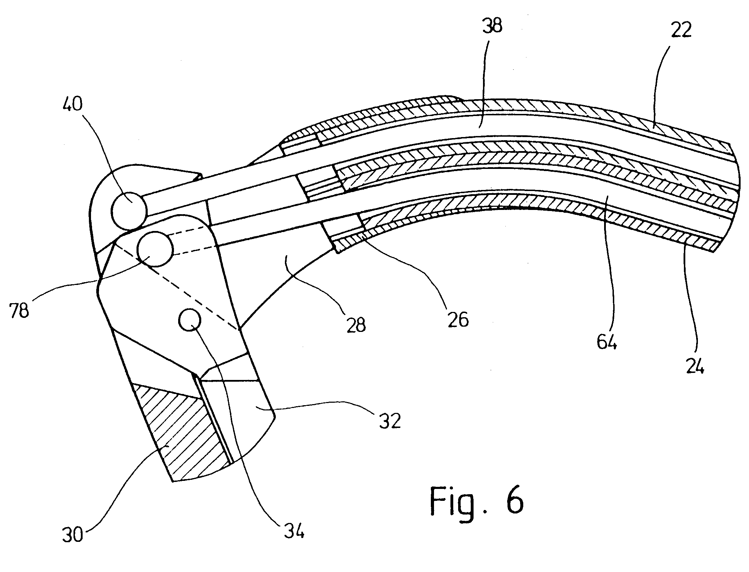

Insbesondere aus der Darstellung von Fig. 6 ist zu entnehmen, daß ja das mit dem Draht 38 verbundene Maulteil 30 am Maulteil 32 geschlossen liegt, somit diesem nicht voreilen kann.

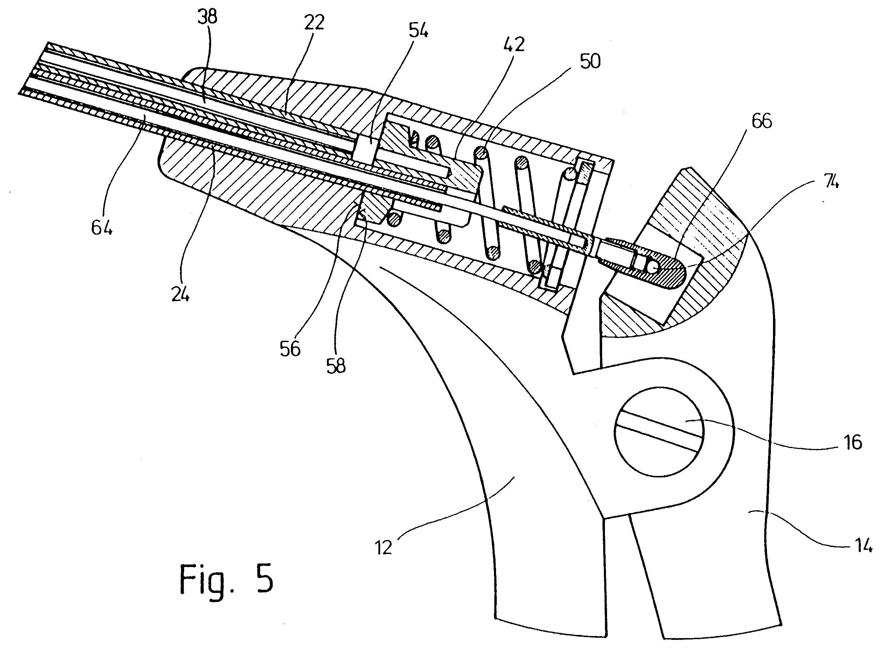

Diese gemeinsame Verschwenkung von Maulteil 30 und 32 geschlossen aneinanderliegend erfolgt solange, bis das Endstück 42 mit seiner Stirnfläche 58 auf die Bodenfläche 46 der Aussparung 44 trifft. Diese Situation ist in Fig. 5 dargestellt. Ein weiteres Vorschieben des Drahtes 38 ist nunmehr blockiert, d.h. auch das Maulteil 30 ist nunmehr vor einer weiteren Verschwenkung entgegen des Uhrzeigersinns blockiert.

Bei einer weiteren Öffnungsbewegung des Griffelementes 14 kann jedoch der Draht 64 durch das Endstück 42 hindurch weiter in Richtung patientennahes Ende verschoben werden, wodurch dann das Maulteil 32 relativ zum ortsfest blockierten Maulteil 30 weiterbewegt bzw. um den Achszapfen 34 verschwenkt wird, so daß die Maulteile 30, 32 sich voneinander wegbewegen, also geöffnet werden.

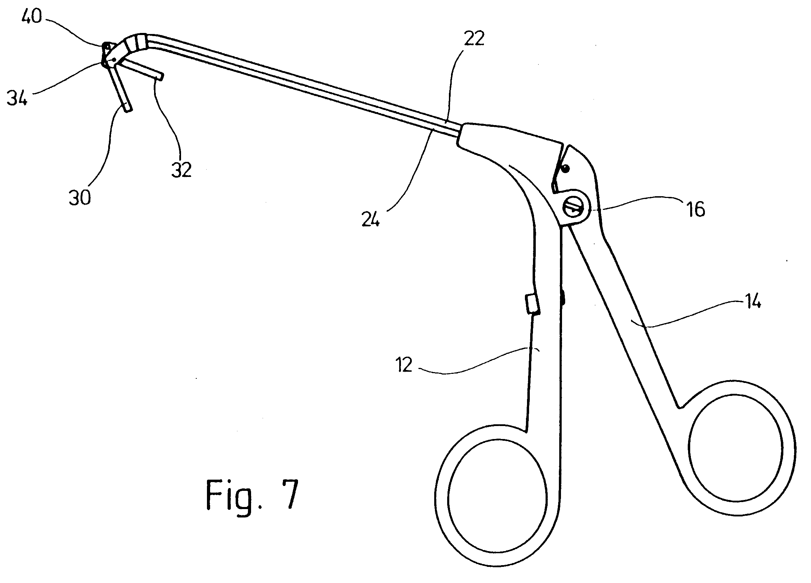

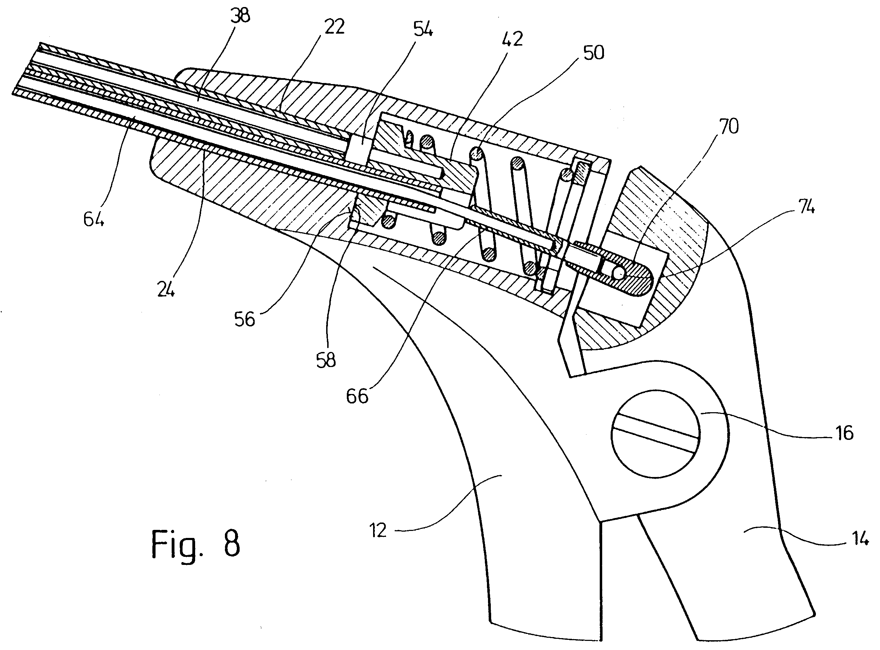

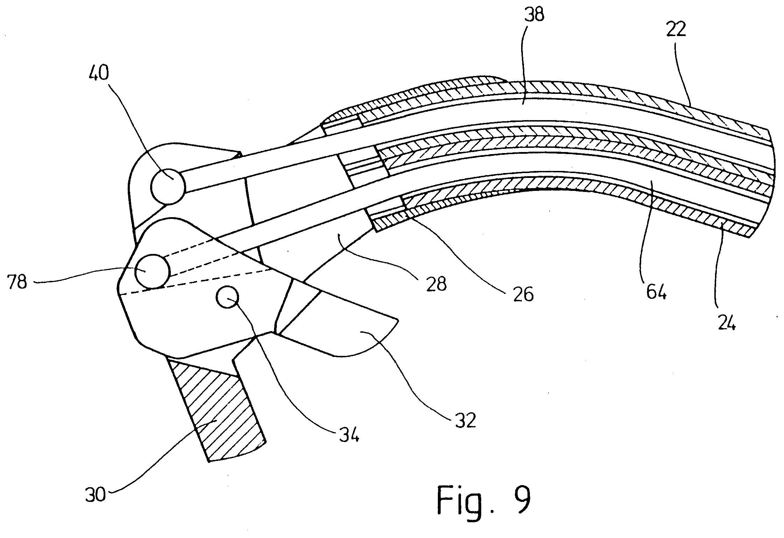

Eine derartige Situation ist in den Fig. 7 bis 9 dargestellt. In dieser dritten Betriebsstellung sind die Griffelemente 12 und 14 nahezu maximal weit geöffnet, wobei insbesondere aus Fig. 8 zu entnehmen ist, daß dies gerade noch soweit möglich ist, bis das obere Ende des Griffelementes 14 auf das entsprechend gegenüberliegende Ende des Griffelementes 12 trifft.

In diesem Stellung mit geöffneten Maulteilen 30, 32 ist es nun möglich, zwischen diesen ein zu erfassendes Gewebeteil zu bringen und durch Schließen der Griffelemente 12 und 14 mit dem damit verbundenen Schließen der Maulteile 30 und 32 dieses Gewebeteil zu fassen.

Die Schließbewegung der Maulteile 30 und 32 erfolgt so lange, bis der Widerstandsdruck des zwischen den Maulteilen 30 und 32 aufgenommenen Gewebestückes größer ist als die Kraft der Feder 52. Wird dies überschritten, führt ein weiteres Schließen der Griffelemente 12 und 14 dazu, daß das Endstück 42 aus der in Fig. 8 dargestellten Position nach rechts verschoben wird, also sich von dem blockierenden Sitz löst, wodurch eine gemeinsame Verschwenkung der Maulteile 30 und 32 um den Achszapfen 34 im Uhrzeigersinn möglich ist. Die Kraft der Feder 50 sorgt dafür, daß die Maulteile 30, 32 mit entsprechender Kraft geschlossen gehalten werden, somit das dazwischen erfaßte Gewebestück derart festgehalten ist, daß dieses, aufgrund der Verschwenkbewegung, von der Stelle abgetrennt wird, von der es entnommen wird. Durch weiteres Schließen der Griffelemente 12 und 14 können dann die Maulteile 30 und 32 in eine Stellung gebracht werden, die ein möglichst problemloses Abziehen der medizinischen Zange von der Entnahmestelle ermöglicht.

Um an Ort und Stelle den Abtrennvorgang möglichst genau beobachten zu können, ist es möglich, die medizinische Zange 10 mit einem Endoskop zu verbinden. Dazu ist es möglich, neben die Rohre 22 und 24 einen Endoskopschaft zu legen und soweit vorzuschieben, daß dessen Endbereich im Bereich des Endstückes 26 zum Liegen kommt, so daß über das Endoskop die Entnahmestelle und die Verschwenkbewegung der Maulteile 30, 32 exakt beobachtet und dann gesteuert werden kann. Es ist möglich, die Zange 10 zugleich als integraler Bestandteil eines Endoskopes auszubilden, oder zunächst die Zange einzuführen und anschließend das Endoskop längs der Rohre 22 und 24 geführt nachzuschieben.

## Patentansprüche

1. Medizinische Zange, mit zwei relativ zueinander bewegbaren Griffelementen (12, 14) am patientenfernen Ende, durch die zwei bewegbar am patientennahen Ende angeordnete Maulteile (30, 32) über ein Betätigungselement (62) relativ zueinander bewegbar sind, und bei der beide Maulteile (30, 32) gemeinsam, ohne Relativbewegung zueinander verschwenkbar sind, dadurch gekennzeichnet, daß über das Betätigungselement (62) in einem ersten Bewegungsbereich der Griffelemente (12, 14) beide Maulteile (30, 32) gemeinsam verschwenkbar sind, und daß in einem zweiten Bewegungsbereich der Griffelemente (12, 14) ein Maulteil (32) relativ zum anderen Maulteil (30) bewegbar ist.

2. Medizinische Zange nach Anspruch 1, dadurch gekennzeichnet, daß beide Maulteile (30, 32) um eine gemeinsame Achse (34) verschwenkbar am patientennahen Ende angeordnet sind.

3. Medizinische Zange nach Anspruch 1 oder 2, dadurch gekennzeichnet, daß im zweiten Bewegungsbereich der Griffelemente (12, 14) ein Maulteil (30) blockiert ist und das andere Maulteil (32) relativ zum blockierten Maulteil (30) bewegbar ist.

4. Medizinische Zange nach einem der Ansprüche 1 bis 3, dadurch gekennzeichnet, daß das Betätigungselement (62) einerseits mit einem Griffelement (14) verbunden ist, und andererseits mit dem Maulteil (32) gelenkig verbunden ist, das auch im zweiten Bewegungsbereich bewegbar ist.

5. Medizinische Zange nach einem der Ansprüche 1 bis 4, dadurch gekennzeichnet, daß das Maulteil (30), das im zweiten Bewegungsbereich ortsfest blockiert ist, im ersten Bewegungsbereich gegen die Kraft einer Feder (50) von der Blockierstellung weg in Richtung gemeinsamer Verschwenkung der Maulteile (30, 32) bewegbar ist, und daß diese Federkraft in der entgegensetzten Richtung die gemeinsame Bewegung beider Maulteile (30, 32) zusammen unterstützt.

6. Medizinische Zange nach Anspruch 5, dadurch gekennzeichnet, daß das Maulteil (30), das ortsfest blockierbar ist, gelenkig mit einem mit einer Feder (50) beaufschlagten Steuerelement (36) verbunden ist, das an dem Griffelement (12) beweglich gelagert ist, das nicht mit dem Betätigungselement (62) verbunden ist.

7. Medizinische Zange nach Anspruch 6, dadurch gekennzeichnet, daß das Steuerelement (36) am patientenfernen Ende mit einem Endstück (42) versehen ist, auf das die Feder (50) einwirkt.

8. Medizinische Zange nach einem der Ansprüche 1 bis 7, dadurch gekennzeichnet, daß die Zange (10) am patiennahen Ende gekrümmt verläuft.

9. Medizinische Zange nach einem der Ansprüche 1 bis 8, dadurch gekennzeichnet, daß sie als integraler Bestandteil eines Endoskopes ausgebildet ist.

## Claims

1. Medical forceps with two handle elements (12, 14), which can be moved relative to one another, disposed at the end remote from the patient, by means of which two jaw parts (30, 32) disposed at the end nearest the patient can be moved relative to one another via an actuation element (62), and wherein both jaw parts (30, 32) can be pivoted together without a relative movement one to another, characterized in that, via the actuation element (62), in a first movement range of the handle elements (12, 14) both jaw parts (30, 32) can be pivoted together, and, in a second movement range of the handle elements (12, 14), one jaw part (32) can be moved relative to the other jaw part (30).

2. Medical forceps according to claim 1, characterized in that both jaw parts (30, 32) are arranged pivotedly about a common shaft (34) at the end nearest the patient.

3. Medical forceps according to claims 1 or 2, characterized in that in the second movement range of the handle elements (12, 14) one jaw part (30) is immobilized, and the other jaw part (32) can move relative to the immobilized jaw part (30).

4. Medical forceps according to anyone of claims 1 through 3, characterized in that the actuation element (62) is connected at one end to a handle element (14) and at the other end is connected in an articulated fashion to the jaw part (32) that can also be moved in the second movement range.

5. Medical forceps according to anyone of claims 1 through 4, characterized in that the jaw part (30) that is immobilized in a fixed position in the second movement range can be moved, in the first movement range, against the force of a spring (50) away from the immobilized position toward shared pivoting of the jaw parts (30, 32); and in the opposite direction this spring force supports the shared movement of the two jaw parts (30, 32) together.

6. Medical forceps according to claim 5, characterized in that the jaw part (30) that can be immobilized in a fixed position is connected in an articulated fashion to a control element (36), acted upon by a spring (50), which is movably mounted on the handle element (12) that is not connected to the actuation element (62).

7. Medical forceps according to claim 6, characterized in that the control element (36), at the end remote from the patient, is provided with an end piece (42) on which the spring (50) acts.

8. Medical forceps according to anyone of claims 1 through 7, characterized in that the forceps (10) is curved at the end nearest the patient.

9. Medical forceps according to anyone of claims 1 through 8, characterized in that the forceps is configured as an integral component of an endoscope.

## Revendications

1. Pince médicale, avec deux éléments formant poignée (12, 14) mobiles l'un par rapport à l'autre à l'extrémité distante du patient, par lesquelles deux parties formant bouche (30, 32) placées mobiles à l'extrémité proche du patient peuvent être déplacées relativement l'une à l'autre par l'intermédiaire d'un élément d'actionnement (62), et dans laquelle les deux parties formant bouche (30, 32) peuvent pivoter en commun sans déplacement relatif l'une par rapport à l'autre, caractérisée en ce que les deux parties formant bouche (30, 32) peuvent pivoter en commun par l'intermédiaire de l'élément d'actionnement (62) dans une première plage de déplacement des éléments formant poignée (12, 14), et en ce que, dans une deuxième plage de déplacement des éléments formant poignée (12, 14), une partie formant bouche (32) est mobile par rapport à l'autre partie formant bouche (30).

2. Pince médicale selon la revendication 1, caractérisée en ce que les deux parties formant bouche (30, 32) sont montées pivotantes autour d'un axe commun (34) à l'extrémité proche du patient.

3. Pince médicale selon la revendication 1 ou 2, caractérisée en ce que, dans la deuxième plage de déplacement des éléments formant poignée (12, 14), une partie formant bouche (30) est bloquée et l'autre partie formant bouche (32) est mobile par rapport à la partie formant bouche (30) qui est bloquée.

4. Pince médicale selon l'une des revendications 1 à 3, caractérisée en ce que l'élément d'actionnement (62) est relié d'une part à un élément formant poignée (14), et est relié d'autre part de façon articulée à la partie formant bouche (32) qui est également mobile dans la deuxième plage de déplacement.

5. Pince médicale selon l'une des revendications 1 à 4, caractérisée en ce que la partie formant bouche (30), qui est bloquée immobile dans la deuxième plage de déplacement, est mobile dans la première plage de déplacement contre la force d'un ressort (50) depuis la position de blocage dans la direction du pivotement commun des parties formant bouche (30, 32), et en ce que cette force de ressort assiste dans la direction opposée le mouvement commun des deux parties formant bouche (12, 14).

6. Pince médicale selon la revendication 5, caractérisée en ce que la partie formant bouche (30), qui peut être bloquée immobile, est reliée de façon articulée à un élément de commande (36) sur lequel est monté un ressort (50) et qui est monté mobile sur l'élément formant poignée (12) qui n'est pas relié à l'élément d'actionnement (62).

7. Pince médicale selon la revendication 6, caractérisée en ce que l'élément de commande (36) est muni à l'extrémité distante du patient d'un embout (42) sur lequel agit le ressort (50).

8. Pince médicale selon l'une des revendications 1 à 7, caractérisée en ce que la pince (10) s'étend de manière courbe à l'extrémité proche du patient.

9. Pince médicale selon l'une des revendications 1 à 8, caractérisée en ce qu'elle est conformée en élément solidaire d'un endoscope.
